Europäisches Patentamt

European Patent Office

Office européen des brevets

(11) Veröffentlichungsnummer: **0 149 103**
**A2**

(12) **EUROPÄISCHE PATENTANMELDUNG**

(21) Anmeldenummer: 84114784.6

(22) Anmeldetag: 05.12.84

(51) Int. Cl.⁴: **H 05 G 1/64**
**G 21 K 1/02, A 61 B 6/06**

(30) Priorität: 19.12.83 DE 3345871

(43) Veröffentlichungstag der Anmeldung:
24.07.85 Patentblatt 85/30

(84) Benannte Vertragsstaaten:
DE FR

(71) Anmelder: Siemens Aktiengesellschaft
Berlin und München Wittelsbacherplatz 2
D-8000 München 2(DE)

(72) Erfinder: Balfanz, Rainer
Im Heuschlag 17
D-8520 Erlangen(DE)

(72) Erfinder: Beiküfner, Georg
Kiefernschlag 1
D-8505 Röthenbach(DE)

(72) Erfinder: Gall, Arthur
Fichtenstrasse 1
D-8521 Langensendelbach(DE)

(54) Röntgendiagnostikanlage mit einer Bildverstärker-Fernsehkette und Indirektkamera.

㊼ Die Erfindung betrifft eine Röntgendiagnostikanlage mit einer Bildverstärker-Fernsehkette (5 bis 8) und Indirektkamera (6a). Am Gehäuse des Röntgenbildverstärkers (5) ist vor dessen Eingangsleuchtschirm (10) eine Irisblende (11) befestigt, durch die das von Strahlung getroffene Feld des Eingangsleuchtschirmes (10) einstellbar ist. Dadurch wird die das Röntgenbild verschlechternde Auswirkung von Streustrahlung gemindert.

EP 0 149 103 A2

0149103

Siemens Aktiengesellschaft      Unser Zeichen
Berlin und München      VPA **83** P 34 22 **E**

Röntgendiagnostikanlage mit einer Bildverstärker-Fernsehkette und Indirektkamera

Es sind Röntgendiagnostikanlagen mit einer Bildverstärker-Fernsehkette und Indirektkamera bekannt, bei denen durch eine Primärstrahlenblende an der Röntgenröhre das von Primärstrahlung getroffene Feld des Eingangsleuchtschirmes des Röntgenbildverstärkers einblendbar ist. Da der Eingangsleuchtschirm jedoch in seiner Gesamtheit frei liegt, trifft auf denjenigen Bereichen, von denen die Primärstrahlung ausgeblendet ist, die insbesondere vom Aufnahmeobjekt ausgehende Streustrahlung auf. Diese Streustrahlung führt zu Leuchterscheinungen am Eingangsleuchtschirm und damit auch zur Freisetzung von Elektronen durch die Fotokathode des Röntgenbildverstärkers. Dadurch wird einerseits das von der Fernsehkamera und der Indirektkamera am Ausgangsleuchtschirm des Röntgenbildverstärkers aufgenommene Bild von einem nur durch die Streustrahlung erzeugten äußeren Bereich umgeben. Andererseits wird auch der störende Bilduntergrund erhöht.

Der Erfindung liegt die Aufgabe zugrunde, eine Röntgendiagnostikanlage mit einer Bildverstärker-Fernsehkette und Indirektkamera so auszubilden, daß die Auswirkung der Streustrahlung auf das Röntgenbild gegenüber dem Stand der Technik verringert wird.

Diese Aufgabe ist erfindungsgemäß dadurch gelöst, daß am Gehäuse des Röntgenbildverstärkers vor dessen Eingangsleuchtschirm eine Blende befestigt ist, durch die das von Strahlung getroffene Feld des Eingangsleucht-

Tp 2 Ler / 25.11.1983

0149103

schirmes einstellbar ist. Diese Blende kann synchron mit der Primärstrahlenblende in der Weise eingestellt werden, daß nur jeweils dasjenige Feld auf dem Eingangsleuchtschirm des Röntgenbildverstärkers der Strahlung ausgesetzt wird, das durch die Primärstrahlenblende gewählt ist. Dadurch wird das Einwirken von Streustrahlung auf die Randbereiche des Eingangsleuchtschirmes des Röntgenbildverstärkers, die häufig nicht zur eigentlichen Bildgebung beitragen, reduziert und insgesamt eine wesentliche Bildverbesserung erreicht.

Die Blende kann zweckmäßigerweise eine Irisblende sein, die den Durchmesser des vor dem Eingangsleuchtschirm des Röntgenbildverstärkers eingeblendeten Feldes an das durch die Primärstrahlenblende eingeblendete Feld anpaßt.

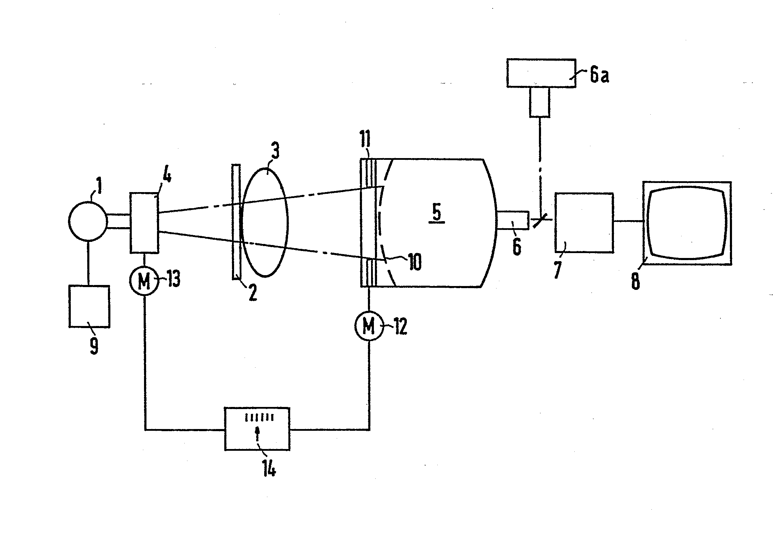

Die Erfindung ist nachfolgend anhand eines in der Zeichnung dargestellten Ausführungsbeispieles näher erläutert.

In der Zeichnung ist eine Röntgenröhre 1 dargestellt, die einen auf einer Liege 2 liegenden Patienten 3 durchstrahlt. Das jeweilige Strahlenfeld wird durch eine einstellbare Primärstrahlenblende 4 festgelegt. Für die Bilderfassung ist ein Röntgenbildverstärker 5 mit nachgeschalteter Fernsehkamera 6 sowie Indirektkamera 6a vorgesehen. Das von der Fernsehkamera 6 erzeugte Videosignal wird über eine Fernsehzentrale 7 auf einem Monitor 8 als Röntgenbild wiedergegeben bzw. von der Indirektkamera 6a aufgezeichnet. Die Röntgenröhre 1 wird von einem Röntgengenerator 9 gespeist.

Am Gehäuse des Röntgenbildverstärkers 5 ist vor dessen Eingangsleuchtschirm 10 eine Irisblende 11 befestigt, durch die das von Strahlung getroffene Feld des Eingangsleuchtschirmes 10 einstellbar ist. Die Irisblende 11 ist durch einen Elektromotor 12 einstellbar. Für die Einstellung der Primärstrahlenblende 4 dient ein Elektromotor 13.

Die Einstellung der Blenden 4 und 11 erfolgt durch eine gemeinsame Einstellvorrichtung 14, durch die die Motoren 12 und 13 gemeinsam so ansteuerbar sind, daß jeweils nur ein Eingangsfeld solcher Größe auf dem Eingangsleuchtschirm 10 des Röntgenbildverstärkers 5 eingeblendet wird, daß diejenigen Teile des Eingangsleuchtschirmes 10, die nicht von Primärstrahlung getroffen werden, abgeblendet werden.

2 Patentansprüche
1 Figur

Patentansprüche

1. Röntgendiagnostikanlage mit einer Bildverstärker-Fernsehkette (5 bis 8) und Indirektkamera (6a), d a d u r c h   g e k e n n z e i c h n e t ,  daß am Gehäuse des Röntgenbildverstärkers (5) vor dessen Eingangsleuchtschirm (10) eine Blende (11) befestigt ist, durch die das von Strahlung getroffene Feld des Eingangsleuchtschirmes (10) einstellbar ist.

2. Röntgendiagnostikanlage nach Anspruch 1,  d a - d u r c h   g e k e n n z e i c h n e t ,  daß die Blende eine Irisblende (11) ist.